# EUROPEAN PATENT SPECIFICATION

(11) **EP 1 658 890 B1**
(45) Date of publication and mention of the grant of the patent: **28.05.2008**
(21) Application number: 05025455.6
(22) Date of filing: 22.11.2005
(51) Int. Cl.: B01F 13/08, B81B 1/00, C12N 1/06

(54) **Microfluidic device including microchannel on which plurality of electromagnets are disposed, and methods of mixing sample and lysing cells using the microfluidic device**
Mikrofluidische Vorrichtung mit einem Mikrokanal mit mehreren Elektromagneten, und Verfahren zur Mischung von Proben und zur Zellyse durch die Verwendung der mikrofluidischen Vorrichtung
Dispositif microfluidique avec microcanal comprenant plusieurs électro-aimants, et méthode pour mélanger des échantillons et pour lyser de cellules en utilisant ledit dispositif microfluidique

(30) Priority: 23.11.2004 KR 2004096152
(43) Date of publication of application: 24.05.2006
(73) Proprietor: Samsung Electronics Co., Ltd., Suwon-si, Gyeonggi-Do (KR)
(72) Inventor: Kim, Jin-tae, Taen-eub Hwaseong-si Gyeonggi-do (KR); Oh, Kwang-wook 211-1504 Sinyeongtong, Hwaseong-si Gyeonngi-do (KR); Cho, Yoon-kyoung, Yeongtong-gu Suwon-si Gyeonggi-do (KR); Peak, Sang-hyun, Yangcheon-gu Seoul (KR); Kim, Sook-young c/o Samsung Advanced Institute of Technology, Yongin-si Gyeonggi-do (KR); Park, Chin-sung, Giheung-eub Yongin-si Gyeonggi-do (KR); Namkoong, Kak, Songpa-gu Seoul (KR)
(74) Representative: Grünecker, Kinkeldey, Stockmair & Schwanhäusser Anwaltssozietät

(56) References cited:
- DE-C1- 4 243 692
- US-A1- 2004 114 458
- US-B1- 6 632 662
- SUZUKI H ET AL: "A magnetic force driven chaotic micro-mixer" PROCEEDINGS OF THE IEEE 15TH. ANNUAL INTERNATIONAL CONFERENCE ON MICROELECTRO MECHANICAL SYSTEMS. MEMS 2002. LAS VEGAS, NV, JAN. 20 - 24, 2002, IEEE INTERNATIONAL MICRO ELECTRO MECHANICAL SYSTEMS CONFERENCE, NEW YORK, NY : IEEE, US, vol. CONF. 15, 2002, pages 40-43, XP010577590 ISBN: 0-7803-7185-2
- R. RONG, J.W. CHOI, C. H. AHN: "A novel magnetic chaotic mixer for in-flow mixing of magnetic beads" 7TH INTERNATIONAL CONFERENCE ON MINIATURIZED CHEMICAL AND BIOCHEMICAL ANALYSIS SYSTEMS, OCTOBER 5-9, 2003; UTAS 2003, SQUAW VALLEY, CALIFORNIA USA, 2003, pages 335-338, XP002377920 ISBN: 0-9743611-0-0

## Description

The present invention is directed to a microfluidic device and a method of mixing a fluidity sample containing a magnetic material using a microfluidic device in accordance with the preambles of claims 1 and 5.

A device and a method of this type are disclosed in Hiroaki Suzuki and Chih-ming Ho "A Magnetic Force Driven Chaotic Micro-Mixer". The micromixer includes a mixing region comprising a microchannel and a plurality of contact pads arranged on opposite sides of the microchannel. The contact pads of opposite sides are of opposite poles, so that the magnetic flux crosses the channel diametrically.

R Rong, J. W. Choi and C. H. Ahn "A Novel Magnetic Chaotic Mixer for In-Flow Mixing of Magnetic Beads" describes a micromixing unit driven by three magnetic pole pairs excited with electromagnets coupled to magnetic pole tips using through-hole vias. The microfluidic channels are fabricated in close proximity to the pole tips. The driving electromagnets and the actuating tips are on opposite sides of the wafer containing the microchannel.

US-A-2004/0114458 describes a device for mixing fluids comprising a chamber containing the liquids to be mixed. A ferromagnetic core is centred in the chambers and magnetic means are suited to create a magnetic field rotating around the ferromagnetic core.

Fluids do not mix well in microchannels of microfluidic devices because they form a laminar flow, and various attempts have been made to solve this problem. For example, a method of increasing a contact area between the two fluids at which diffusion occurs by using a complex 2- or 3-dimensional structure and a method of using a magnetic field have been developed. However, the manufacturing process for producing the channels required for such methods is complex and the stream of fluids may be blocked when the channels have a complex 2- or 3-dimensional structure. In addition, a mixing zone in which mixing occurs outside of the channels through which the fluids flow is required, and thus an additional dead volume is produced.

An example of a conventional method of mixing fluids in a container using a magnetic field is disclosed in U.S. Patent No. 6,467,946, which teaches a method of mixing a liquid solution in a container having a false bottom. The method includes placing a ferromagnetic material into the liquid solution contained in the container, and rotating a pair of magnetic fields in a circular pattern in close proximity to the container near the location of the false bottom. However, in this method, bar-shaped electromagnets must be connected to an external motor to be rotated, and it is difficult to synchronize a pair of electric fields.

Therefore, a method which can easily and efficiently mix fluids within microchannels using a magnetic field is still required.

### SUMMARY OF THE INVENTION

The present invention according to claim 1 provides a microfluidic device including a microchannel on which electromagnets are disposed.

The present invention according to claim 5 also provides a method of efficiently mixing samples including a magnetic material using the microfluidic device.

The present invention also provides a method of efficiently lysing cells using the microfluidic device.

According to an aspect of the present invention, there is provided a microfluidic device comprising at least one inlet, at least one outlet, and a microchannel connecting the inlet and the outlet and two or more electromagnets disposed on sidewalls of the microchannel and oriented in a predetermined direction with respect to the direction in which the microchannel extends.

According to another aspect of the present invention, there is provided a method of mixing a sample containing a magnetic material using a microfluidic device including at least one inlet, at least one outlet, a microchannel connecting the inlet and the outlet, and two or more electromagnets disposed on sidewalls of the microchannel and oriented in a predetermined direction with respect to the direction in which the microchannel extends, the method comprising: injecting the sample containing the magnetic material into the microchannel via the inlet of the microfluidic device; and operating the electromagnets disposed on the sidewalls of the microchannel simultaneously or in cycles.

According to another aspect of the present invention, there is provided a method of lysing cells using the microfluidic device of the present invention, the method comprising: injecting a magnetic bead and the cells into the microchannel via the inlet of the microfluidic device; and lysing the cells by operating the two or more electromagnets disposed on the sidewalls of the microchannel.

### BRIEF DESCRIPTION OF THE DRAWINGS

The above and other features and advantages of the present invention will become more apparent by describing in detail exemplary embodiments thereof with reference to the attached drawings in which:
FIG. 1 is a diagram illustrating a microfluidic device including two inlet ports, a mixing zone, and an outlet port according to an embodiment of the present invention;
FIG. 2 is a diagram illustrating a microchannel with electromagnets extending perpendicular to sidewalls of the microchannel according to an embodiment of the present invention;
FIG. 3 is schematic diagram of a micro mixing unit with four electromagnets disposed on a crossing line formed by a plane intersecting a microchannel perpendicularly to the direction in which the microchannel extends according to an embodiment of the present invention;
FIG. 4 is a diagram illustrating a process of turning on the electromagnets of the micro mixing unit illustrated in FIG. 3 in clockwise cycles when seen from where a sample flows from;
FIG. 5 is a diagram illustrating the movement of a magnetic material according to the change in a magnetic field when turning on the electromagnets in the clockwise cycles when seen from where the sample flows from, as in FIG. 4;
FIG. 6 is a diagram illustrating the change in velocity of the sample as a result of the movement of the magnetic material when the electromagnets are turned on in the clockwise cycles when seen from where the sample flows from, as in FIG. 4;
FIG. 7 is a diagram illustrating a microchannel in which four sets of four electromagnets are oriented perpendicularly to the direction in which the microchannel extends, and the electromagnets are turned on in cycles according to an embodiment of the present invention; and
FIG. 8 is a diagram illustrating a microchannel in which four sets of four electromagnets are oriented perpendicularly to the direction in which the microchannel extends, and the sets of electromagnets are alternately turned on in the clockwise and counterclockwise directions according to an embodiment of the present invention.

### DETAILED DESCRIPTION OF THE INVENTION

According to an aspect of the present invention, there is provided a microfluidic device comprising at least one inlet, at least one outlet, and a microchannel connecting the inlet and the outlet and two or more electromagnets disposed on sidewalls of the microchannel and oriented in a predetermined direction with respect to the direction in which the microchannel extends.

The term "microfluidic device" refers to a device including at least one inlet and at least one outlet which are connected via microchannels. The microfluidic device can include a micro chamber in which a chemical reaction occurs or is analyzed. In the present invention, a cross section of the microchannel may be of any shape, for example, circular, rectangular, or trapezoidal.

In the microfluidic device according to the present invention described above, at least two electromagnets are arranged on sidewalls of the microchannel that are oriented in a predetermined direction with respect to the direction in which the microchannel extends. The term "electromagnet" used in the present invention may refer to any electromagnet known to one of ordinary skill in the art. The electromagnet can be composed of a power supply, a coil connected to the power supply, and a core around which the coil is wrapped. The electromagnets may be disposed at any angle with respect to the direction in which the microchannel extends as long as the electromagnets operate in cycles and a rotating magnetic field is provided within the microchannel. The electromagnets may be oriented substantially at right angles to the direction in which the microchannel extends, or at right angles. In the present invention, the phrase "electromagnets are disposed on the sidewalls of the microchannels" refers to electromagnets contacting the sidewalls of the microchannels as well as near the microchannels to provide a magnetic field within the microchannels.

In the present invention, the electromagnet may be disposed in an electromagnet set composed of a plurality of electromagnets on a crossing line formed by a plane intersecting the microchannel at a predetermined angle with respect to the direction in which the microchannel extends. Four electromagnets may be disposed on a crossing line formed by a plane intersecting the microchannel at a right angle with respect to the direction in which the microchannel extends.

A plurality of sets of electromagnets may be formed on each of a plurality of crossing lines formed by planes intersecting the microchannel at a predetermined angle with respect to the direction in which the microchannel extends. For example, the microfluidic device may include four electromagnets disposed on each of the four crossing lines.

Alternatively, the electromagnets may be disposed on the sidewalls of the microchannel in a spiral along the direction in which the microchannel extends.

According to another aspect of the present invention, there is provided a method of mixing a sample containing a magnetic material using a microfluidic device including at least one inlet, at least one outlet, a microchannel connecting the inlet and the outlet, and two or more electromagnets disposed on sidewalls of the microchannel and oriented in a predetermined direction with respect to the direction in which the microchannel extends, the method comprising: injecting the sample containing the magnetic material into the microchannel via the inlet of the microfluidic device; and operating the electromagnets disposed on the sidewalls of the microchannel simultaneously or in cycles.

The method includes injecting the sample containing the magnetic material into the microchannel via the inlet of the microfluidic device. The magnetic material may be contained in biological samples such as blood or tissue, or cells, or antigen, or antibody, or DNA, or enzyme, or chemical samples, or may be provided to the solution artificially. The magnetic material may be any material having a magnetic property, for example, but not limited to, one selected from the group consisting of iron, nickel, chrome, and an oxide thereof. The magnetic material may be formed in microbeads or nanobeads, but is not limited to these forms. In the injecting of the sample, the magnetic material and the sample may be simultaneously injected or a mixture of the sample and the magnetic material may be injected through a single inlet. Alternatively, the sample and the magnetic material may be injected through different inlets and be mixed at any location in the microfluidic device.

The method includes mixing the sample containing the magnetic material by operating simultaneously or in cycles two or more electromagnets disposed on the sidewalls of the microchannel of the microfluidic device. When the electromagnets are simultaneously operated, the two or more electromagnets may be disposed in a spiral on the outer sidewalls of the microchannel with respect to the direction in which the microchannel extends. In the present invention, the phrase "the electromagnets operate in cycles" denotes one electromagnet being turned on/off in regular intervals in an electromagnet set composed of two or more electromagnets.

In the microfluidic device used in the method, the electromagnets may be disposed vertically with respect to the direction in which the microchannel extends.

In the microfluidic device used in the method, the electromagnets may be disposed on a crossing line formed by a plane intersecting the microchannel at a predetermined angle with respect to the direction in which the microchannel extends.

In the microfluidic device used in the method, four electromagnets may be disposed on a crossing line formed by a plane intersecting the microchannel at right angles with respect to the direction in which the microchannel extends.

In the microfluidic device used in the method, a plurality of electromagnets may be formed on each of a plurality of crossing lines.

In the microfluidic device used in the method, the four electromagnets may be disposed on four crossing lines.

In the microfluidic device used in the method, the electromagnets may be disposed on the sidewall of the microchannel in a spiral along the direction in which the microchannel extends.

In the method according to the present invention, a ligand material may be immobilized to the magnetic material, and a molecule which can specifically bind with the ligand material may be included in the sample containing the magnetic material. The ligand material may be, but is not limited to, a ligand, an antigen, enzyme substrate or an inhibitor. The molecules which specifically bind to the ligand material may be, but are not limited to, a receptor, an antibody, and an antigen.

According to another aspect of the present invention, there is provided a method of lysing cells using a microfluidic device. The method includes: injecting a magnetic bead and the cells into the microchannel via the inlet of the microfluidic device; and lysing the cells by operating the two or more electromagnets disposed on the sidewalls of the microchannel.

The microfluidic device used in the present method is the same as the microfluidic device described above.

The present invention will now be described more fully with reference to the accompanying drawings, in which exemplary embodiments of the invention are shown.

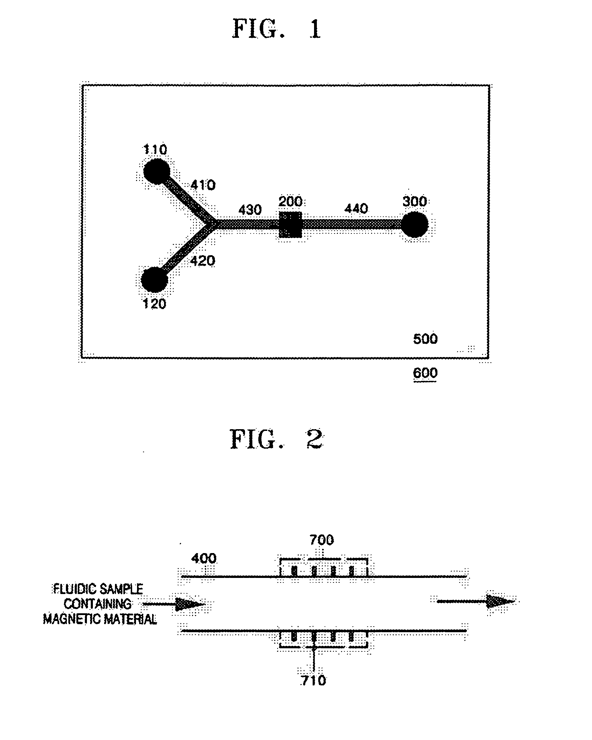

FIG. 1 is a diagram illustrating a microfluidic device including two inlet ports 110 and 120, a mixing zone 200, and an outlet port 300 according to an embodiment of the present invention. A fluidic sample is injected into the microfluidic device via the inlet ports 110 and 120, flows to the mixing zone 200 via inlet microchannels 410 and 420 and a microchannel 430, and is discharged from the mixing zone 200 to the outside through the outlet port 300 via a microchannel 440. In the microfluidic device, at least two electromagnets (not shown) are disposed on sidewalls of each of the inlet microchannels 410 and 420 and the microchannels 430 and 440. The electromagnets are oriented in predetermined directions with respect to the directions in which the inlet microchannels 410 and 420 and the microchannels 430 and 440 extend.

FIG. 2 is a diagram illustrating a microchannel 400 with electromagnets 710 oriented perpendicular to sidewalls of the microchannel 400 according to an embodiment of the present invention. As illustrated in FIG. 2, the microchannel 400 includes a micro mixing unit 700 where the electromagnets 710 on the sidewall of the microchannel 400 are oriented perpendicular to the direction in which a fluidic sample flows. When a fluidic sample containing a magnetic material exists in the microchannel 400 or flows through the microchannel 400, the micro mixing unit 700 applies a magnetic field to the magnetic material to mix the fluidic sample using the magnetic field. Such mixing can be performed by turning on the electromagnets 710 disposed on the sidewall of the microchannel 400 simultaneously or in cycles so that the fluidic sample containing the magnetic material is affected by the magnetic force. The electromagnets 710 may be turned on/off so that a rotating magnetic force is applied to the fluidic sample as the electromagnets 710 operate in cycles along the circumference of the sidewall of the microchannel 400. The electromagnets 710 may be turned on/off using a method well known to those of ordinary skill in the art. For example, the sequential operation of the electromagnets 710 may be performed using a switch which regulates power supplied to the electromagnets 710 in cycles.

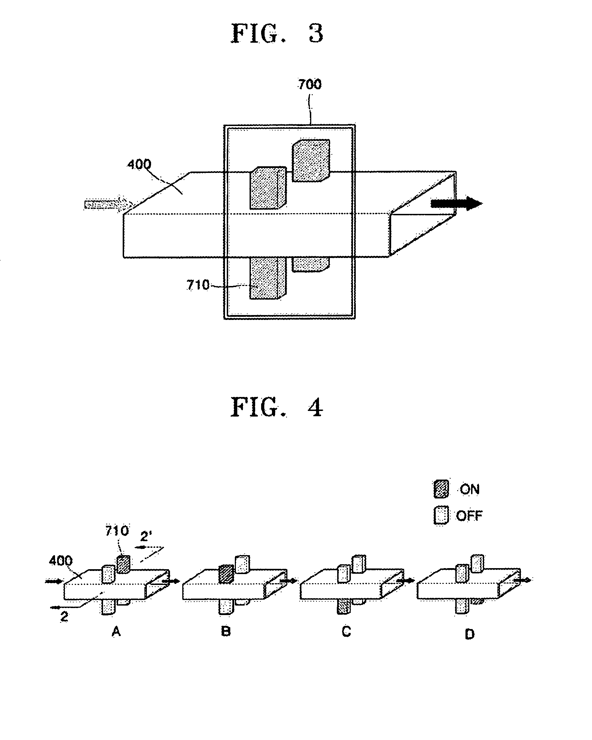

FIG. 3 is schematic diagram of a micro mixing unit 700 in which four electromagnets 710 are disposed on a crossing line formed by a plane intersecting a microchannel 400 perpendicularly to the direction in which the microchannel extends according to an embodiment of the present invention. Referring to FIG. 3, the cross section of the microchannel 400 is rectangular. However, the cross section of the microchannel 400 is not limited to being rectangular, and may be any shape.

FIG. 4 is a diagram illustrating a process of turning on the electromagnets 710 of the micro mixing unit 700 in clockwise cycles when seen from where a fluidic sample flows from. As illustrated in FIG. 4, the electromagnets 710, which operate as time passes from A through D, operate in clockwise cycles.

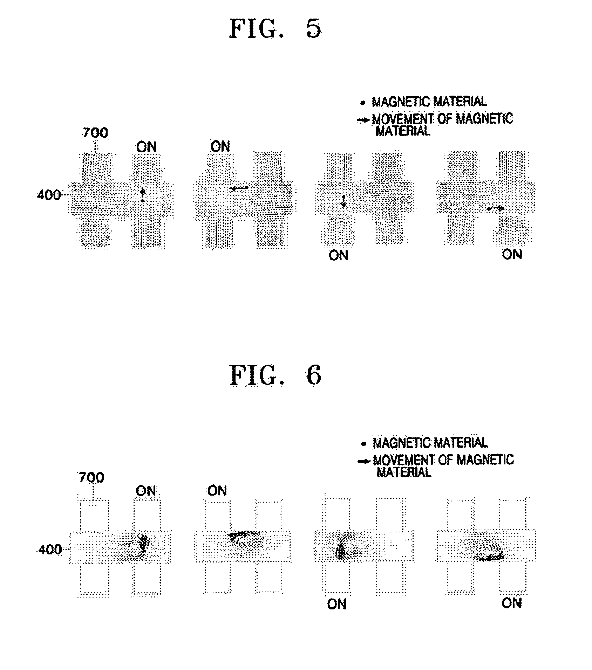

FIG. 5 is a diagram illustrating the movement of a magnetic material according to the change in a magnetic field when the electromagnets 710 are turned on in clockwise cycles when seen from where the fluidic sample flows from, as in FIG. 4. As illustrated in FIG. 5, it can be seen that the magnetic material inside the microchannel 400 rotates as the magnetic field rotates when the electromagnets 710 are turned on/off in cycles.

FIG. 6 is a diagram illustrating the change in velocity of the fluidic sample as a result of the movement of the magnetic material when the electromagnets 710 are turned on in clockwise cycles when seen from where the fluidic sample flows from, as in FIG. 4. As illustrated in FIG. 6, it can be seen that the fluidic sample within the microchannel 400 rotates and mixes as the magnetic field rotates when the electromagnets 710 are turned on/off in cycles.

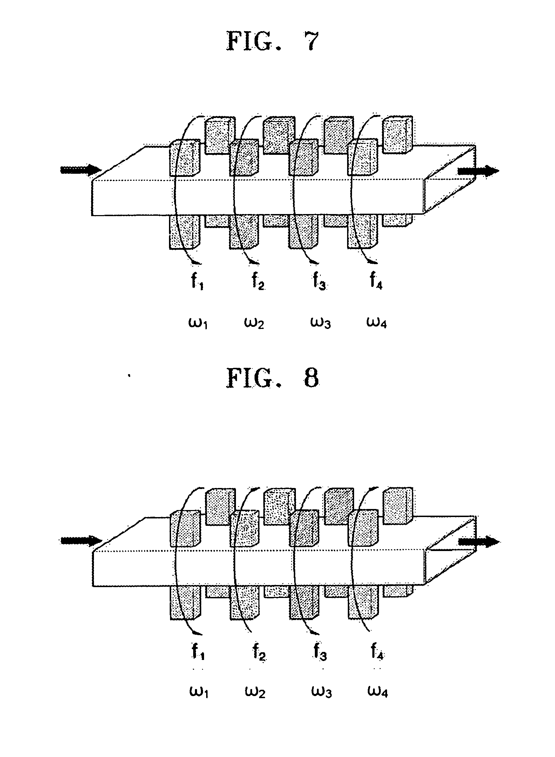

FIG. 7 is a diagram illustrating a microchannel in which four sets of four electromagnets are oriented perpendicularly to the direction in which the microchannel extends, and the electromagnets are turned on in cycles according to an embodiment of the present invention. Referring to FIG. 7, each of the electromagnet sets can be turned on/off in cycles. The frequencies f₁, f₂, f₃, and f₄ of the on/off cycles and angular velocities ω₁, ω₂, ω₃, and ω₄ of the magnetic fields produced by the electromagnets may be the same or different.

FIG. 8 is a diagram illustrating a microchannel in which four sets of four electromagnets are oriented perpendicularly to the direction in which the microchannel extends, and the sets of electromagnets are alternately turned on in the clockwise and counterclockwise directions according to an embodiment of the present invention. Referring to FIG. 8, each of the sets of electromagnets can be turned on/off in cycles so that a set of electromagnets turns on when the adjacent set of electromagnets is "off" and vice versa. The frequencies f₁, f₂, f₃, and f₄ of the on/off cycles and angular velocities ω₁, ω₂, ω₃, and ω₄ of the magnetic fields produced by the electromagnets may be the same or different.

According to a microfluidic device of the present invention, a fluidic sample containing a magnetic material that flows through a microchannel of the microfluidic device can be efficiently mixed.

According to a method of mixing a fluidic sample containing a magnetic material using the microfluidic device, the fluidic sample can be efficiently mixed inside a microchannel.

According to a method of lysing cells using the microfluidic device, the cells can be efficiently lysed inside a microchannel using a magnetic field.

While the present invention has been particularly shown and described with reference to exemplary embodiments thereof, it will be understood by those of ordinary skill in the art that various changes in form and details may be made therein without departing from the scope of the present invention as defined by the following claims.

## Claims

1. A microfluidic device comprising:
at least one inlet (110, 120);
at least one outlet (300);
a microchannel (410, 420, 430, 440) connecting the inlet and the outlet; and
a micro mixing unit (700) for applying a magnetic field to magnetic material contained in a fluid sample to be mixed,
**characterized in that** the micro mixing unit (700) includes
two or more electromagnets (710) having a core and a coil disposed on sidewalls of the microchannel (410, 420, 430, 440) on a crossing line formed by a plane intersecting the microchannel and oriented in a direction perpendicular with respect to the direction in which the microchannel extends.

2. The microfluidic device of claim 1, wherein there are four electromagnets (710) disposed on a crossing line formed by a plane intersecting the microchannel at a right angle with respect to the direction in which the microchannel extends.

3. The microfluidic device of claim 1, wherein a plurality of electromagnets (710) are formed on each of a plurality of crossing lines.

4. The microfluidic device of claim 3, wherein the four electromagnets are disposed on each of four crossing lines.

5. A method of mixing a fluidic sample containing a magnetic material using a microfluidic device including at least one inlet (110, 120), at least one outlet (300), a microchannel (410, 420, 430, 440) connecting the inlet and the outlet, and a micro mixing unit (700) for applying a magnetic field to magnetic material contained in the fluid sample to be mixed,
the method comprising injecting the fluidic sample containing the magnetic material into the microchannel via the inlet of the microfluidic device; and
operating the micro mixing unit (700) for applying the magnetic field,
**characterized in that** the micro mixing unit (700) comprises two or more electromagnets (710) having a core and a coil disposed on sidewalls of the microchannel on a crossing line formed by a plane intersecting the microchannel in a direction perpendicular with respect to the direction in which the microchannel extends, the method comprising:
operating the electromagnets (710) disposed on the sidewalls of the microchannel simultaneously or in cycles.

6. The method of claim 5, wherein, in the microfluidic device, there are four electromagnets (710) disposed on a crossing line formed by a plane intersecting the microchannel at a right angle with respect to the direction in which the microchannel extends.

7. The method of claim 5, wherein, in the microfluidic device, a plurality of electromagnets (710) are formed on each of a plurality of crossing lines.

8. The method of claim 7, wherein, in the microfluidic device, the four electromagnets (710) are disposed on each of four crossing lines.

9. The method of claim 5, wherein, the injecting of the fluidic sample comprises injecting the fluidic sample containing the magnetic material and a solution to be mixed into the microchannel through different inlets (110, 120).

10. The method of claim 5, wherein a ligand is immobilized in the magnetic material, and a molecule which can specifically bind with the ligand is included in the fluidic sample containing the magnetic material.

11. A method of lysing cells using the microfluidic device of any of Claims 1 through 4, the method comprising:
injecting a magnetic bead and the cells into the microchannel via the inlet of the microfluidic device; and
lysing the cells by operating the two or more electromagnets (710) disposed on the sidewalls of the microchannel.

## Patentansprüche

1. Mikrofluidische Vorrichtung mit:
mindestens einem Einlass (110, 120),
mindestens einem Auslass (300),
einem Mikrokanal (410, 420, 430, 440), der den Einlass und den Auslass verbindet, und
eine Mikromischeinheit (700) zum Anlegen eines magnetischen Feldes an ein magnetisches Material, das in einer zu mischenden Strömungsmittelprobe enthalten ist,
**dadurch gekennzeichnet, dass** die Mikromischeinheit (700) zwei oder mehr Elektromagnete (710) mit einem Kern und einer Spule enthält, die an Seitenwänden des Mikrokanals (410, 420, 430, 440) an einer querenden Linie angeordnet sind, die gebildet wird durch eine den Mikrokanal schneidende Ebene und die in einer Richtung orientiert sind, die rechtwinklig bezüglich der Richtung verläuft, in die sich der Mikrokanal erstreckt.

2. Mikrofluidische Vorrichtung nach Anspruch 1, wobei vier Elektromagnete (710) vorgesehen sind, die an einer querenden Linie angeordnet sind, die gebildet wird durch eine Ebene, die den Mikrokanal unter einem rechten Winkel bezüglich der Richtung, in der sich der Mikrokanal erstreckt, schneidet.

3. Mikrofluidische Einrichtung nach Anspruch 1, wobei eine Vielzahl von Elektromagneten (710) an jeder von einer Vielzahl querenden Linien ausgebildet sind.

4. Mikrofluidische Einrichtung nach Anspruch 3, wobei die vier Elektromagneten an jeder der vier querenden Linien angeordnet sind.

5. Verfahren zum Mischen einer Strömungsmittelprobe, die ein magnetisches Material enthält, unter Verwendung einer mikrofluidischen Vorrichtung, die mindestens einen Einlass (110, 120), mindestens einen Auslass (300), einen Mikrokanal (410, 420, 430, 440), der den Einlass und den Auslass verbindet und eine Mikromischeinheit (700) zum Aufbringen eines Magnetfeldes auf magnetisches Material, das in der zu mischenden Strömungsmittelprobe enthalten ist, enthält,
wobei das Verfahren das Injizieren der Strömungsmittelprobe, die das magnetische Material enthält, in den Mikrokanal über den Einlass der mikrofluidischen Einrichtung, und
den Betrieb der Mikromischeinheit (700) zum Aufbringen des magnetischen Feldes umfasst,
**dadurch gekennzeichnet, dass** die Mikromischeinheit (700) zwei oder mehr Elektromagnete (710) mit einem Kern und einer Spule umfasst, die an Seitenwänden des Mikrokanals an einer querenden Linie angeordnet sind, die durch eine Ebene gebildet ist, die den Mikrokanal in einer Richtung rechtwinklig bezüglich der Richtung, in die sich der Mikrokanal erstreckt, schneidet, wobei das Verfahren den gleichzeitigen oder zyklischen Betrieb der Elektromagnete umfasst, die an den Seitenwänden des Mikrokanals angeordnet sind.

6. Verfahren nach Anspruch 5, wobei in der mikrofluidischen Vorrichtung vier Elektromagnete (710) vorgesehen sind, die auf einer querenden Linie angeordnet sind, die durch eine Ebene gebildet wird, die den Mikrokanal unter einem rechten Winkel bezüglich der Richtung, in der sich der Mikrokanal erstreckt, schneidet.

7. Verfahren nach Anspruch 5, wobei bei der mikrofluidischen Vorrichtung eine Vielzahl von Elektromagneten (710) an jeder von einer Vielzahl von querenden Linien ausgebildet sind.

8. Verfahren nach Anspruch 7, wobei in der mikrofluidischen Vorrichtung die vier Elektromagnete (710) an jeder von vier querenden Linien angeordnet sind.

9. Verfahren nach Anspruch 5, wobei das Einspritzen der Strömungsmittelprobe das Einspritzen der Strömungsmittelprobe, die das magnetische Material enthält, und einer zu mischenden Lösung in den Mikrokanal durch unterschiedliche Einlässe (110, 120) umfasst.

10. Verfahren nach Anspruch 5, wobei ein Ligand im magnetischen Material immobilisiert ist, und in der Strömungsmittelprobe, die das magnetische Material enthält, ein Molekül enthalten ist, das spezifisch an den Liganden anbinden kann.

11. Verfahren für einen Zellaufschluss unter Verwendung der mikrofluidischen Vorrichtung nach einem der Ansprüche 1 bis 4, wobei das Verfahren umfasst:
das Injizieren eines magnetischen Partikels und der Zellen in den Mikrokanal über den Einlass der mikrofluidischen Vorrichtung; und
das Aufschließen der Zellen durch Betätigung der zwei oder mehr Elektromagnete (710), die an den Seitenwänden des Mikrokanals angeordnet sind.

## Revendications

1. Dispositif microfluidique comprenant :
au moins une entrée (110, 120) ;
au moins une sortie (300) ;
un microcanal (410, 420, 430, 440) raccordant l'entrée et la sortie ; et
une unité de micromélange (700) pour appliquer un champ magnétique à un matériau magnétique contenu dans un échantillon de fluide à mélanger,
**caractérisé en ce que** l'unité de micromélange (700) comprend
deux électroaimants (710) ou plus ayant un noyau et une bobine disposés sur les parois latérales du microcanal (410, 420, 430, 440) sur une ligne de croisement formée par un plan croisant le microcanal et orientée dans une direction perpendiculaire par rapport à la direction dans laquelle s'étend le microcanal.

2. Dispositif microfluidique selon la revendication 1, dans lequel il existe quatre électroaimants (710) disposés sur une ligne de croisement formée par un plan croisant le microcanal à angle droit par rapport à la direction dans laquelle s'étend le microcanal.

3. Dispositif microcfluidique selon la revendication 1, dans lequel une pluralité d'électroaimants (710) sont formés sur chacune d'une pluralité de lignes de croisement.

4. Dispositif microfluidique selon la revendication 3, dans lequel les quatre électroaimants sont disposés sur chacune des quatre lignes de croisement.

5. Procédé de mélange d'un échantillon fluidique contenant un matériau magnétique en utilisant un dispositif microfluidique comprenant au moins une entrée (110, 120), au moins une sortie (300), un microcanal (410, 420, 430, 440) raccordant l'entrée et la sortie, et une unité de micromélange (700) pour appliquer un champ magnétique au matériau magnétique contenu dans l'échantillon de fluide à mélanger,
le procédé comprenant l'injection de l'échantillon fluidique contenant le matériau magnétique dans le microcanal via l'entrée du dispositif microfluidique ; et
le fonctionnement de l'unité de micromélange (700) pour appliquer le champ magnétique,
**caractérisé en ce que** l'unité de micromélange (700) comprend deux électroaimants (710) ou plus ayant un noyau et une bobine disposés sur les parois latérales du microcanal sur une ligne de croisement formée par un plan croisant le microcanal dans une direction perpendiculaire par rapport à la direction dans laquelle s'étend le microcanal, le procédé comprenant :
le fonctionnement des électroaimants (710) disposés sur les parois latérales du microcanal de manière simultanée ou en cycles.

6. Procédé selon la revendication 5, dans lequel, dans le dispositif microfluidique, il existe quatre électroaimants (710) disposés sur une ligne de croisement formée par un plan croisant le microcanal à angle droit par rapport à la direction dans laquelle s'étend le microcanal.

7. Procédé selon la revendication 5, dans lequel, dans le dispositif microfluidique, une pluralité d'électroaimants (710) sont formés sur chacune d'une pluralité de lignes de croisement.

8. Procédé selon la revendication 7, dans lequel, dans le dispositif microfluidique, les quatre électroaimants (710) sont disposés sur chacune des quatre lignes de croisement.

9. Procédé selon la revendication 5, dans lequel l'injection de l'échantillon fluidique comprend l'injection de l'échantillon fluidique contenant le matériau magnétique et une solution à mélanger dans le microcanal à travers des entrées différentes (110, 120).

10. Procédé selon la revendication 5, dans lequel un ligand est immobilisé dans le matériau magnétique et une molécule qui peut se lier spécifiquement avec le ligand est incluse dans l'échantillon fluidique contenant le matériau magnétique.

11. Procédé de lyse de cellules utilisant le dispositif microfluidique selon l'une quelconque des revendications 1 à 4, le procédé comprenant :
l'injection d'une perle magnétique et des cellules dans le microcanal via l'entrée du dispositif microfluidique ; et
la lyse des cellules en faisant fonctionner les deux électroaimants (710) ou plus disposés sur les parois latérales du microcanal.
